# EUROPEAN PATENT SPECIFICATION

(11) **EP 0 783 476 B1**
(45) Date of publication and mention of the grant of the patent: **09.12.1998**
(21) Application number: 95935416.8
(22) Date of filing: 28.09.1995
(51) Int. Cl.: C07C 45/58, C07C 45/80, C07C 47/19, C07C 31/20, C07C 29/141

(54) **PROCESS FOR PREPARING 1,3-ALKANEDIOLS AND 3-HYDROXYALDEHYDES**
VERFAHREN ZUR HERSTELLUNG VON 1,3-ALKANDIOLEN UND 3-HYDROXYALDEHYDEN
PROCEDE DE PREPARATION DE 1,3-ALCANEDIOLS ET DE 3-HYDROXYALDEHYDES

(30) Priority: 30.09.1994 US 316660; 30.09.1994 US 316669; 30.09.1994 US 316677; 30.09.1994 US 316679; 30.09.1994 US 316680
(43) Date of publication of application: 16.07.1997
(73) Proprietor: SHELL INTERNATIONALE RESEARCH MAATSCHAPPIJ B.V., 2596 HR Den Haag (NL)
(72) Inventor: ARHANCET, Juan, Pedro, Katy, TX 77450 (US); FORSCHNER, Thomas, Clayton, Richmond, TX 77469 (US); POWELL, Joseph, Broun, Houston, TX 77070 (US); SEMPLE, Thomas, Carl, Friendswood, TX 77546 (US); SLAUGH, Lynn, Henry, Houston, TX 77070 (US); THOMASON, Terry, Blane, Houston, TX 77077 (US); WEIDER, Paul, Richard, Houston, TX 77083 (US); ALLEN, Kevin, Dale, Katy, TX 77449 (US); EUBANKS, David, Cleve, Houston, TX 77083 (US); FONG, Howard, Lam-Ho, Sugar Lands, TX 77478 (US); JOHNSON, David, William, Richmond, TX 77459 (US); LIN, Jiang, Jen, Houston, TX 77083 (US); MULLIN, Stephen, Blake, Katy, TX 77449 (US)
(86) International application number: EP9503870
(87) International publication number: WO9610552

(56) References cited:
- WO-A-94/18149
- US-A- 4 973 741

## Description

This invention relates to a process for preparing 1,3-alkanediols and 3-hydroxyaldehydes by hydroformylating an oxirane (1,2-epoxide). In particular, the invention relates to a process for preparing 1,3-propanediol by hydroformylating ethylene oxide in the presence of a Group VIII-based hydroformylation catalyst and hydrogenating the hydroformylation product.

The preparation of 1,3-alkanediols like 1,3-propanediol (PDO) is disclosed in US-A-3 687 981. The process comprises the hydroformylation of an oxirane such as ethylene oxide, in a concentration of more than 15 weight percent based on the total liquid reaction mixture, in the presence of a metallic carbonyl catalyst containing a metal from Group VIII, followed by hydrogenation of the hydroformylation product. The hydroformylation product of that process is the cyclic hemiacetal dimer of 3-hydroxy-propanal (HPA), i.e., 2- (2-hydroxyethyl)-4-hydroxy-1, 3-dioxane. PDO is of particular interest as intermediate in the production of polyesters for fibres and films.

Despite the publication of this patent in 1972, fibre-grade polyesters based on PDO are as yet not commercially available. Separation of the catalyst from the cyclic hemiacetal produced in US-A-3 687 981, by phase separation, is complicated and inadequate. As a result, the cost for preparing polymer-grade PDO is too high.

In US-A-3 456 017 and US-A-3 463 819 1,3 alkanediols are prepared directly, with only minor amounts of the intermediate hydroformylation product, in the presence of certain phosphine-modified cobalt carbonyl catalysts. Commercialisation of the process of these US patents is ruled out, due to the excessive amounts of catalyst employed therein. Also in WO 94/18149 phosphine-modified cobalt carbonyl catalysts are used. They are used in a much smaller amount than in the US patents, producing primarily the 3-hydroxyaldehyde. Although activity of the phosphine-modified cobalt-carbonyl catalyst described in the international application is high, improvement remains desirable, particularly in view of the undesirable co-production of acetaldehyde. Besides, the cost of the phosphines, which are notoriously difficult to retain when recycling the catalyst, adversely affect the economy of that process.

It would be desirable to prepare 3-hydroxyaldehydes and 1,3-alkanediols selectively and cheaply. It is therefore an object of the invention to provide an economical process for the preparation of 3-hydroxyaldehydes and 1,3-alkanediols in the presence of a hydroformylation catalyst which process allows for the convenient recycle of the catalyst.

Accordingly, the invention provides a process for preparing 1,3-alkanediols and 3-hydroxyaldehydes by hydroformylating an oxirane with carbon monoxide and hydrogen in the presence of one or more Group VIII metal-based hydroformylation catalysts, which may contain up to 50 mole% based on the metal of phosphine-modified catalysts, and in the presence of an organic solvent, wherein the concentration of the oxirane at the start of the reaction is less than 15 percent by weight (wt%) based on the weight of the total liquid reaction mixture. The process is preferably carried out at a temperature below 100 °C.

As a result, an intermediate product mixture is obtained, essentially composed of starting components and the 3-hydroxyaldehyde, the latter in an amount that is less than 15 wt% based on the total liquid reaction mixture. At this concentration, the selectivity towards the 3-hydroxyaldehyde is high, whereas the catalyst may be conveniently recycled.

The oxirane comprises an organic compound, two carbon atoms of which are connected by an oxy linkage as well as by a carbon-carbon single bond. In general terms, the oxiranes comprise hydrocarbyl-epoxides, having at least 2, preferably up to 30, more preferably up to 20, most preferably up to 10 carbon atoms. The hydrocarbyl group may be aryl, alkyl, alkenyl, aralkyl, cycloalkyl, or even alkylene; straight chain or branched chain. Suitable examples of oxiranes include 1,2-epoxy(cyclo)alkanes, such as ethylene oxide, propylene oxide, 1,2-epoxyoctane, 1,2-epoxycyclohexane, 1,2-epoxy-2,4,4-trimethylhexane and the like, and 1,2-epoxyalkenes such as 1,2-epoxy-4-pentene and the like. Ethylene oxide and propylene oxide are preferred. In view of the demand for PDO, ethylene oxide (EO) is the oxirane most preferably used in the process of the invention.

The hydroformylation reaction is carried out in a liquid solvent inert to the reactants and products, i.e., that is not consumed during the reaction. Upon completion of the reaction, the liquid solvent facilitates the separation of the hydroformylation product. The separation may be carried out by allowing the product to form a separate layer, as is disclosed in US-A-3 687 981. However, as discussed below, it is preferred to carried out the separation by extraction with an aqueous liquid. In general, ideal solvents for the hydroformylation process will (a) exhibit low to moderate polarity such that the 3-hydroxyaldehyde will be dissolved to a concentration of at least about 5 wt% under hydroformylation conditions, while significant solvent will remain as a separate phase upon extraction with the aqueous liquid, (b) dissolve carbon monoxide, and (c) be essentially non-water-miscible. By "essentially non-water-miscible" is meant that the solvent has a solubility in water at 25 °C of less than 25 wt% so as to form a separate hydrocarbonrich phase upon extraction of the 3-hydroxyaldehyde from the hydroformylation reaction mixture. Preferably, this solubility is less than 10 wt%, most preferably less than 5 wt%. The solubility of carbon monoxide in the selected solvent will generally be greater than 0.15 v/v (1 atm, 25 °C), preferably greater than 0.25 v/v, expressed in terms of Ostwald coefficients.

The preferred class of solvents are alcohols and ethers which can be described according to the formula (1)

R¹-O-R² (1)

in which R¹ is selected from hydrogen, a linear, branched, cyclic or aromatic C₁₋₂₀ hydrocarbyl or mono-or polyalkylene oxide and R² is selected from a linear, branched, cyclic or aromatic C₁₋₂₀ hydrocarbyl, alkoxy or mono- or polyalkylene oxide, or R¹, R² and O together form a cyclic ether. The most preferred hydroformylation solvents can be described by the formula (2) 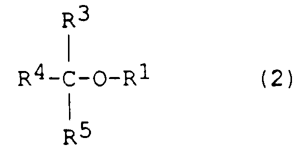 in which R¹ is selected from hydrogen or a C₁₋₈ hydrocarbyl and R³, R⁴ and R⁵ are independently selected from a C₁₋₈ hydrocarbyl, alkoxy or mono- or polyalkylene oxide. Such ethers include, for example, tetrahydrofuran, methyl-t-butyl ether, ethyl-t-butyl ether, ethoxyethyl ether, phenylisobutyl ether, diethyl ether, diphenyl ether, and diisopropyl ether. Blends of solvents such as t-butylalcohol/hexane, tetrahydrofuran/toluene and tetrahydrofuran/heptane can also be used to achieve the desired solvent properties. The currently preferred solvent, because of the high yields of HPA which can be achieved under moderate reaction conditions, is methyl-t-butyl ether.

The hydroformylation reaction is conducted in the presence of any metallic carbonyl hydroformylation catalyst, as long as less than 50 mole% thereof preferably less than 10 mole% thereof are phosphine-modified. These catalysts are transition metals, particularly those metals of Group VIII of the Periodic Table, e.g., cobalt, iron, nickel, osmium and complexes described, for example, in US-A-3 161 672. Best results, however, are obtained when a cobalt-based catalyst is used, unmodified cobalt carbonyl compounds being preferred.

The cobalt-based catalyst can be supplied to the hydroformylation reactor as a cobalt carbonyl such as dicobaltoctacarbonyl or cobalt hydridocarbonyl. It may also be supplied in essentially any other form including metal, supported metal, Raney-cobalt, hydroxide, oxide, carbonate, sulfate, acetylacetonate, salt of a fatty acid, or aqueous cobalt salt solution. If not supplied as cobalt carbonyl, operating conditions should be adjusted such that cobalt carbonyls are formed, for instance via reaction with H₂ and CO as described in J. Falbe, "Carbon Monoxide in Organic Synthesis," Springer-Verlag, NY (1970). Typically, these conditions will include a temperature of at least 50 °C and a carbon monoxide partial pressure of at least 0.8 MPa (100 psig). For more rapid reaction, temperatures of 120 to 200 °C should be employed, at CO pressures of at least 3.5 MPa (500 psig). Addition of high surface area activated carbons or zeolites, especially those containing or supporting platinum or palladium metal, is known to accelerate the formation of cobalt carbonyls.

The catalyst is preferably maintained under a stabilising atmosphere of carbon monoxide, which also provides protection against exposure to oxygen. The most economical and preferred catalyst activation and reactivation (of recycled catalyst) method involves converting the cobalt salt (or derivative) under H₂/CO in the presence of the catalyst promoter employed for hydroformylation. The conversion of Co²⁺ to the desired cobalt carbonyl is carried out at a temperature within the range of 75 to 200 °C, preferably 100 to 140 °C and a pressure within the range of 7.0 to 34.6 MPa (1000 to 5000 psig) for a time preferably less than about 3 hours. The preforming step can be carried out in a pressurised preforming reactor or in-situ in the hydroformylation reactor.

The amount of Group VIII metal present in the reaction mixture will vary depending upon the other reaction conditions, but will generally fall within the range of 0.01 wt% to 1 wt%, preferably 0.05 to 0.3 wt%, based on the weight of the reaction mixture.

The hydroformylation reaction mixture will preferably include a catalyst promoter to accelerate the reaction rate. The promoter will generally be present in an amount within the range of 0.01 to 0.6 moles per mole of Group VIII metal.

Suitable promoters include sources of mono- and multivalent metal cations of weak bases such as alkali, alkaline earth and rare earth metal salts of carboxylic acids. Suitable metal salts include sodium, potassium and caesium acetates, propionates and octoates; calcium carbonate and lanthanum acetate. The currently preferred metal salt is sodium acetate.

Also suitable are lipophilic promoters such as lipophilic mono- or dihydroxyarenes, lipophilic tertiary amines or arsines, or lipophilic phosphine oxides respectively arsine oxides, which accelerate the rate of hydroformylation without imparting hydrophilicity (water solubility) to the active catalyst. As used herein, "lipophilic" means that the promoter tends to remain in the organic phase after extraction of HPA with water.

Suitable lipophilic mono- or dihydroxyarenes include those represented by formulae (3) and (4):

C₆R₅OH (3) C₆R₄(OH)₂ (4)

in which each R group is independently selected from hydrogen, a halide, a linear, branched, cyclic or aromatic C₁₋₂₅ hydrocarbyl, alkoxy or mono- or polyalkylene oxide, or in which two or more R groups together form a ring structure. Examples include phenol, nonylphenol, methylphenol, butylphenol, isopropylphenol, 2,2-bis(4-hydroxyphenyl)propane, naphthol, hydroquinone, catechol, dihydroxynaphthalenes and dihydroxyanthracenes. Excellent results have been achieved with phenol and nonylphenol, which are hence preferred.

Suitable lipophilic amines and arsines include those represented by formulae (5) and (6):

NR'₃ (5) AsR'₃ (6)

in which each R' group is independently selected from a linear, branched, cyclic and aromatic C₁₋₂₅ hydrocarbyl, alkoxy or mono- or polyalkylene oxide, or in which two or more of the R' groups together form a ring structure. Such arsines include triphenylarsine and triethylarsine. Examples in which two or more of the R' groups together form a ring structure include pyridine and substituted pyridines described by formula (7): 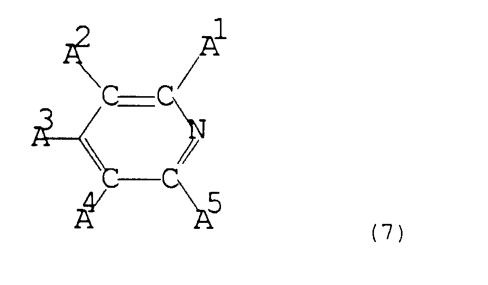 in which each of the A groups is independently selected from hydrogen or a linear, branched, cyclic or aromatic C₁₋₂₅ hydrocarbyl, two or more of which may form a ring structure. Substituted pyridines wherein A¹ and A⁵ are both bulky groups, such as t-butyl, are not preferred. The lipophilic tertiary amine is preferably a non-chelating amine of conjugate acid having a pKa in the range of 5 to 11. Such lipophilic tertiary amines include dimethyldodecylamine, pyridine, 4-(1-butylpentyl)pyridine, quinoline, isoquinoline, lipdine and quinaldine. The preferred amine is nonylpyridine.

Suitable phosphine oxides and arsine oxides include those represented by formulae (8) and (9):

O=PR"₃ (8) O=AsR"₃ (9)

in which each R" group is independently selected from a halide, a linear, branched, cyclic or aromatic C₁₋₂₅ hydrocarbyl, alkoxy or mono- or polyalkylene oxide, or in which two or more R groups together form a ring structure. Such phosphine oxides include triphenylphosphine oxide, tributylphosphine oxide, dimethylphenylphosphine oxide and triethylphosphine oxide. The currently preferred phosphine oxide is triphenylphosphine oxide.

It is generally preferred to regulate the concentration of water in the hydroformylation reaction mixture, as excessive amounts of water reduce the selectivity towards the 1,3-alkanediols and 3-hydroxyaldehydes below acceptable levels and may induce formation of a second liquid phase. At low concentrations, water can assist in promoting the formation of the desired cobalt carbonyl catalyst species. Acceptable water levels will depend upon the solvent used, with more polar solvents generally more tolerant of higher water concentrations. For example, optimum water levels for hydroformylation in methyl-t-butyl ether solvent are believed to be within the range of 1 to 2.5 wt%.

The hydrogen and carbon monoxide will generally be introduced into the reaction vessel in a molar ratio within the range of 1:2 to 8:1, preferably 1:1.5 to 5:1.

The reaction is carried out under conditions effective to produce a hydroformylation reaction mixture comprising a major portion of the 3-hydroxyaldehyde and a minor portion of by-product, if any. Moreover, the level of 3-hydroxyaldehyde in the reaction mixture is preferably maintained at less than 15 wt%, preferably 5 to 10 wt%. (To provide for solvents having different densities, the concentration of 3-hydroxyaldehyde in the reaction mixture can be expressed in molarity, i.e., less than 1.5M, preferably within the range of 0.5 to 1M.).

Suitable, the reaction is carried out at a oxirane concentration that is less than 12 wt%.

Generally, the hydroformylation reaction is carried out at elevated temperature less than 100 °C, preferably 60 to 90 °C, most preferably 75 to 85 °C, and at a pressure within the range of 3.5 to 34.6 MPa (500 to 5000 psig), preferably (for process economics) 7.0 to 24.2 MPa (1000 to 3500 psig), with higher pressures generally imparting greater selectivity. The concentration of 3-hydroxyaldehyde in the intermediate product mixture can be controlled by regulation of process conditions such as oxirane concentration, catalyst concentration, reaction temperature and residence time. In general, relatively low reaction temperatures (below 100 °C) and relatively short residence times within the range of 20 minutes to 1 hour are preferred.

In the practice of the invention method, it is possible to achieve 3-hydroxyaldehyde yields (based on the oxirane conversion) of greater than 80%. For instance, with the hydroformylation of EO in the presence of a cobalt carbonyl, formation of more than 7 wt% HPA in the dilute hydroformylation product mixture, at rates greater than 30 h⁻¹ are achievable. (Catalytic rates are referred to herein in terms of "turnover frequency" or "TOF" and are expressed in units of moles per mole of cobalt per hour, or h⁻¹.) Reported rates are based on the observation that, before a majority of the oxirane, here EO, is converted, the reaction is essentially zero-order in EO concentration and proportional to cobalt concentration.

As mentioned above, separation of the hydroformylation product mixture is carried out economically most attractively by extraction with an aqueous liquid.

Preferably the aqueous liquid is water. The amount of water added to the hydroformylation reaction product mixture will generally be such as to provide a weight ratio of water:mixture within the range of 1:1 to 1:20, preferably 1:5 to 1:15. The addition of water at this stage of the reaction may have the additional advantage of suppressing formation of undesirable heavy ends.

Extraction with a relatively small amount of water provides an aqueous phase which is greater than 20 wt% 3-hydroxyaldehyde, preferably greater than 35 wt% 3-hydroxyaldehyde, permitting economical hydrogenation of the 3-hydroxyaldehyde to the 1,3-alkanediol. The water extraction is preferably carried out at a temperature within the range of 25 to 55 °C, with higher temperatures avoided to minimise condensation products (heavy ends) and catalyst disproportionation to inactive, watersoluble Group VIII metal (e.g., cobalt) compounds. In order to maximise catalyst recovery discussed supra, it is preferred to perform the water extraction under 0.5 to 1.5 MPa (50 to 200 psig) of carbon monoxide at 25 to 55 °C.

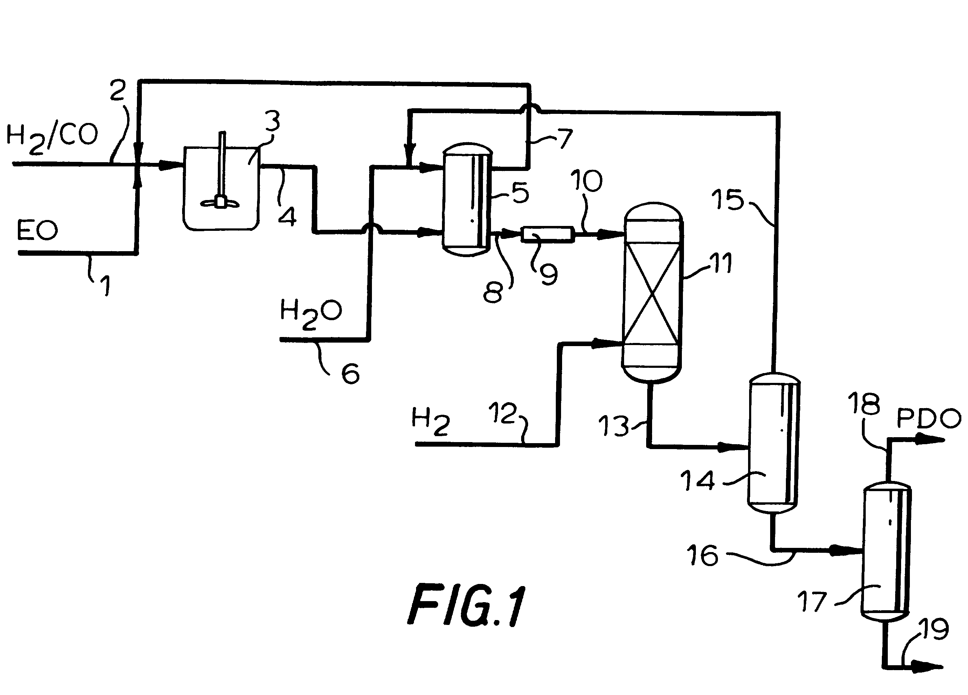

The invention process can be conveniently described by reference to Figure 1. By way of example, the hydroformylation of EO as oxirane will be described. Separate or combined streams of EO *(1)*, carbon monoxide and hydrogen *(2)* are charged to hydroformylation vessel *(3)*, which can be a pressure reaction vessel such as a bubble column or agitated tank, operated batch-wise or in a continuous manner. The feed streams are contacted in the presence of an unmodified cobalt-based catalyst, i.e., a cobalt carbonyl compound which has not been prereacted with a phosphine ligand.

Following the hydroformylation reaction, the hydroformylation reaction product mixture *(4)* containing HPA, the reaction solvent, PDO, the cobalt catalyst and a minor amount of reaction by-products, is passed to extraction vessel *(5)*, to which an aqueous liquid, generally water and optionally a miscible solvent, are added via *(6)* for extraction and concentration of the HPA for the subsequent hydrogenation step. Liquid extraction can be effected by any suitable means, such as mixer-setters, packed or trayed extraction columns or rotating disk contactors. Extraction can if desired be carried out in multiple stages. The water-containing hydroformylation reaction product mixture can be passed to a settling tank (not shown) for resolution into aqueous and organic phases.

The organic phase containing the reaction solvent and the major portion of the cobalt catalyst can be recycled from the extraction vessel to the hydroformylation reaction via *(7)*. Aqueous extract *(8)* is optionally passed through one or more acid ion exchange resin beds *(9)* for removal of any cobalt catalyst present, and the decobalted aqueous product mixture *(10)* is passed to hydrogenation vessel *(11)* and reacted with hydrogen *(12)* in the presence c a hydrogenation catalyst to produce a hydrogenation product mixture *(13)* containing PDO. The hydrogenation step may also revert some heavy ends to PDO. The solvent and extractant water *(15)* can be recovered by distillation in column *(14)* and recycled to the water extraction process via a further distillation (not shown) for separation and purge of light ends. PDO-containing stream *(16)* can be passed to one or more distillation columns *(17)* for recovery of PDO *(18)* from heavy ends *(19)*.

The process of the invention permits the selective and economical synthesis of PDO at moderate temperatures and pressures without the use of a phosphine ligand for the hydroformylation catalyst. The process involves preparation of a reaction product mixture dilute in HPA, then concentration of this HPA by water extraction for subsequent hydrogenation of HPA to PDO.

### Comparative example 1

This experiment illustrates the hydroformylation of ethylene oxide (EO) catalysed by a phosphine-modified cobalt catalyst derived from dicobaltoctacarbonyl.

A 300 ml stirred reactor was charged with 0.87g dicobaltoctacarbonyl, 1.33g bis (1,2-diphenylphosphino)-ethane, 0.125g sodium acetate trihydrate, 0.51g 2-ethylhexanoic acid, and 147.2g "NEODOL" 23 (trademark), a blend of C₁₂ and C₁₃ alcohols. Reactor contents were heated to 165 °C under 1:1 H₂:CO synthesis gas for 1 hour, with agitation at 1000 rpm, to preform the active catalyst. The reactor temperature was decreased to 90 °C, and 20g EO (i.e., 11.8 wt%) were injected via a "blowcase" vessel charged with 10.4 MPa (1500 psig) synthesis gas. The reactor pressure was topped to 10.4 MPa (1500 psig). Reactor pressure decreased over time as a result of hydroformylation of EO substrate. The reactor was refilled to 10.4 MPa (1500 psig) with 1:1 H₂:CO upon a decrease in pressure to 9.1 MPa (1300 psig). In this manner, the uptake of synthesis gas could be monitored as a function of time, to follow the course of the reaction.

Samples of the reaction mixture were periodically withdrawn into chilled n-propanol containing an internal standard (toluene or ethyl acetate) for analysis by capillary gas chromatography (with flame ionisation detector). The analysis indicated an 87% conversion of EO in 3 hours, to give 10 weight percent 3-hydroxypropanal (HPA) intermediate, with some minor hydrogenation to 1,3-propanediol (PDO). This result corresponds to an effective reaction rate of 15 moles of HPA formed per mole of Co catalyst per hour (TOF). Apparent selectivity to acetaldehyde, expressed as the molar ratio of acetaldehyde to the sum of HPA and acetaldehyde, was 27%.

### Example 1

A 300 ml stirred batch reactor was charged under nitrogen with 0.87g dicobaltoctacarbonyl, 1.5g toluene (internal marker) 1.5g undecanol (second marker), and 147g methyl-t-butyl ether (MTBE). The nitrogen atmosphere was flushed with H₂ before the reactor was filled to 8.3 MPa (1200 psi) with 1:1 CO/H₂. Reactor contents were heated to 80 °C for 45 minutes, before injection of 20g EO, with simultaneous increase in reactor pressure to 10.3 MPa (1500 psi) at an H₂/CO ratio of 2.3. The concentration of EO at the start of the reaction was 11.7 wt%. Reactor contents were sampled and analysed. Formation of 2.7 wt% HPA was observed after 30 minutes, for a rate of 20.2 h⁻¹.

### Example 2

The conditions of Example 1 were repeated with addition of 0.5g of dimethyldodecylamine and injection of 12g EO (i.e., 7.4 wt%). Sampling after 45 minutes of reaction indicated formation of 5.7 wt% HPA, for a rate of 31 h⁻¹. This corresponds to a 1.5-fold rate increase over that observed in the absence of promoter. The reaction was continued until formation of 10 wt% HPA at virtual complete conversion of ethylene oxide.

Following reaction, the mixture was cooled to 25 °C and extracted with 30g deionised water under 2.1 MPa (300 psi) CO. The mixture was then transferred to a separation vessel under 0.7 MPa (100 psi CO). Separation yielded 30.75g of a lower aqueous layer containing 24.0 wt% HPA, and an upper organic solvent layer containing 1.0 wt% HPA. Colorimetric analysis of upper and lower layers indicated 94% of the cobalt catalyst to reside in the upper solvent layer, demonstrating separation of a majority of cobalt catalyst from a majority of HPA product.

### Comparative example 2

This experiment illustrates separation of HPA from the cobalt hydroformylation catalyst by distillation. 113.45g of EO hydroformylation reaction product containing 14.32g of HPA intermediate were diluted with 50.lg of tetraethylene glycol dimethylether. The mixture was distilled via a short-path batch still at 10 mm Hg under a slow nitrogen purge at a distillate bottoms temperature ranging from 66 to 108 °C. Distillate fractions were collected and were found by gas chromatographic analysis to contain 6.32g HPA. No HPA was evident in the remaining bottoms sample, which exhibited a significant increase in components heavier than HPA. Total HPA recovery was thus 44% with the remainder degraded to heavy ends.

This experiment demonstrates the problems inherent in thermal separation of highly-reactive HPA intermediate from the reaction mixture. More than half the HPA intermediate was degraded during the separation.

### Example 3

This invention experiment demonstrates separation and concentration of HPA by water extraction. 1507.6g of EO hydroformylation reaction product (MTBE solvent with sodium acetate promoter at 0.2 Na/Co) containing 6.0 wt% HPA intermediate were water extracted at 25 °C under 0.8 MPa (100 psig) nitrogen in a stirred reactor with 298g of deionised water, giving 400.5g of a lower layer containing 20.8 wt% HPA intermediate (3.5-fold concentration). Overall HPA material balance from gas chromatographic analysis of feed, upper phase and lower phase indicated complete recovery of HPA within g.c. experimental error.

The upper layer following water extraction contained 0.14 wt% cobalt, or 65% of the initially-charged catalyst.

This experiment demonstrates the catalyst and product recovery advantages of the invention PDO preparation method. Separation of HPA from the reaction mixture was very efficient and selective. The use of water and low temperatures avoided the degradation of HPA shown in Comparative example 2. The method also allows concentration of HPA for more efficient hydrogenation and final recovery. In addition, a significant fraction (65%) of the cobalt catalyst was readily separated from aqueous HPA product, making efficient recycle of catalyst with reaction solvent possible.

### Example 4

A 300-ml stirred batch reactor was charged under nitrogen with 0.87g dicobaltoctacarbonyl, 1.5g toluene (internal marker), 2g deionised water and 146g MTBE. The nitrogen atmosphere was flushed with H_{2,} and the reactor was filled to 4.2 MPa (600 psig) H₂ and then to 8.4 MPa (1200 psig) with 1:1 CO/H₂. Reactor contents were heated to 80 °C for one hour, and 10g of EO (6.2 wt%) was then injected, with simultaneous increase in reactor pressure to 10.4 MPa (1500 psig) via addition of 1:1 CO/H₂. Reactor contents were sampled and analysed at approximately 40% and nearly 100% conversion of EO, which occurred within two hours. At approximately 40% conversion, 3.3 wt% HPA had been formed at a rate of 18 h⁻¹.

### Example 5

Example 4 was repeated in the absence of added water and with addition of 0.14g of sodium acetate trihydrate as promoter, added at a ratio Na/Co of 0.2. The concentration of EO at the start of the reaction was 6.3 wt%. HPA was formed at a rate of 41 h⁻¹. After cooling and addition of 30g deionised water for extraction, 77% of the cobalt catalyst remained with the upper solvent layer. 23% of the cobalt was extracted with the aqueous product. This fraction corresponds approximately to the amount of sodium acetate added to promote the reaction.

### Examples 6 to 11

These experiments illustrate the effectiveness of lipophilic promoters such as phenol, nonylphenol, hydroquinone, 4-(1-butylpentyl)pyridine, triphenylarsine and triphenylphosphine oxide both to accelerate the hydroformylation reaction and to permit the recycle of essentially all the cobalt catalyst in the organic phase following water extraction of product HPA. Example 4 was repeated with addition of respectively 0.12 g of phenol (Example 6), 0.25g of nonylphenol (7), 0.14g of hydroquinone (8), 0.27g 4-(1-butylpentyl)pyridine (9), 0.4g triphenylarsine (10) or 0.4g triphenylphosphine (11) as promoter, for a ratio of 0.25 moles promoter per mole of cobalt (11 at 0.26) and a concentration of EO at the start of the reaction in the range of 6.2 to 6.3 wt%. Reactor contents were sampled and analysed at approximately 50% conversion and at virtual complete conversion.

Following the reaction, the mixture was cooled to room temperature. About 30g of deionised water were added for extraction of product under 1.5 MPa (200 psig) synthesis gas. After 30 minutes, mixing was terminated and an aqueous product layer containing HPA was isolated. Both layers were analysed.

Results of these experiments are summarised in the Table. From the table it may be learned that the use of a promoter provides a rate increase over that observed in the absence of a promoter in Example 4. Recycle of the cobalt catalyst with the organic layer represents a substantial reduction in cobalt loss relative to that observed with sodium acetate promotion in Example 5.

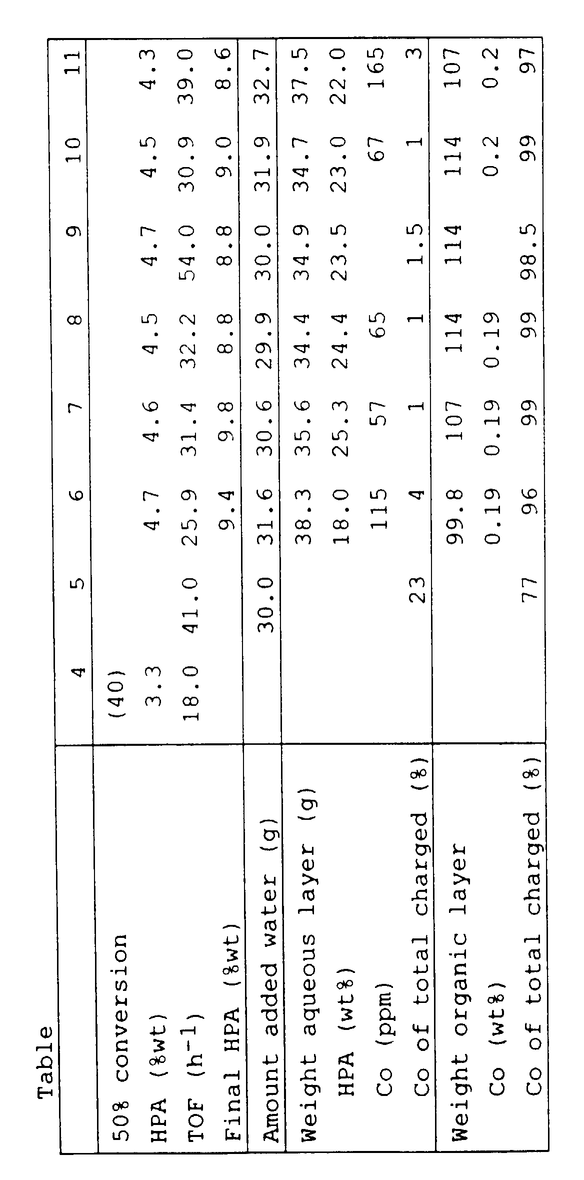

### Example 12

This example illustrates hydrogenation of aqueous HPA obtained from water extraction of the product of EO hydroformylation. 333.4g of extract containing 20 wt% HPA were added to a 500 ml autoclave reactor containing 5.07g of a powdered supported nickel hydrogenation catalyst (Calsicat E-475SR, 50% Ni). The reactor was charged with 7.0 MPa (1000 psig) H₂ and heated to 60 °C for 3 hours. At this time, gas chromatographic analysis indicated 99% conversion of HPA, at 93% selectivity to PDO (moles PDO formed divided by moles HPA consumed) and 3% selectivity to propanol. The reaction temperature was increased to 90 °C for one hour, after which an HPA conversion in excess of 99% was indicated, at an apparent selectivity of 99% PDO and 3.5% propanol. Heating was continued for one additional hour at 110 °C to further improve the selectivity towards PDO by reversal of heavy ends formed during hydroformylation or early hydrogenation.

### Example 13

In order to examine the role of the promoter, a series of reactions was carried out in a small-scale reactor fitted with optics for in situ infrared analysis. In the first reaction, 80 mg (0.234 millimoles) of recrystallised (from CH₂Cl₂) dicobaltoctacarbonyl were added to 17 ml of dried and distilled MTBE in the 30 ml reactor bottom fitted with a ZnS (45) infrared crystal. The top was closed onto the unit and the reactor assembly was removed from the dry box. The inert atmosphere was replaced with carbon monoxide by alternately pressurising the reactor to 1.5 MPa (200 psig) with CO and then depressurising the vessel to atmospheric pressure for a total of 3 cycles. The unit was finally pressurised to 1.5 MPa (200 psig) with CO. The unit was then heated to 80 °C and the pressure in the reactor was adjusted to 2.7 MPa (375 psig) with pure CO. 1.2g (27 millimoles) of EO (i.e., 8.5 wt%) were added to the reactor with hydrogen gas pressure, bringing the total pressure inside the unit to 11.1 MPa (1600 psig) to produce a 3:1; H₂:CO gas cap. Infrared spectra were recorded at 3 minute intervals to monitor the progress of the reaction. The pressure in the unit dropped due to gas consumption and synthesis gas (1:1) was added as required to maintain the total pressure in the reactor between approximately 10.8 and 10.4 MPa (1550 and 1500 psig). A reactor profile of pressure and temperature data was measured digitally via transducer and thermocouple.

The second reaction was carried out in a like manner except that 16 mg (0.096 millimoles) of sodium octoate was also added to the reaction mixture. Again, the concentration of EO at the start of the reaction was 8.5 wt%. The rate of HPA formation was calculated from synthesis gas consumption and checked against the appearance of aldehyde at 1724 cm⁻¹ and the disappearance of the EO band in the infrared spectrum at 870 cm⁻¹. The TOF of the reaction in the absence of a promoter was 15 h⁻¹ and in the presence of sodium octoate the TOF was 41 h⁻¹. At the beginning of the reaction, the infrared spectrum of the catalyst region (2300-2000 cm⁻¹) displayed patterns characteristic of dicobaltoctacarbonyl. The reaction run in the absence of a promoter showed no change in this region of the infrared over the course of the reaction. In contrast, the reaction run with the promoter changes rapidly producing a pattern characteristic of the cobalt acyl complex in addition to the patterns from dicobaltoctacarbonyl. This indicates that the promoter changes the rate determining step in the reaction cycle, resulting in a faster overall reaction rate.

## Claims

1. A process for preparing 1,3-alkanediols and 3-hydroxyaldehydes by hydroformylating an oxirane with carbon monoxide and hydrogen in the presence of one or more Group VIII metal-based hydroformylation catalysts, which may contain up to 50 mole% based on the metal of phosphine-modified catalysts, and in the presence of an organic solvent, wherein the concentration of the oxirane at the start of the reaction is less than 15 percent by weight (wt%) based on the weight of the total liquid reaction mixture.

2. A process as claimed in claim 1, wherein the concentration of the oxirane is less than 12 wt%.

3. A process as claimed in claims 1 or 2, wherein the oxirane is a hydrocarbyl-epoxide having of 2 up to 30 carbon atoms.

4. A process as claimed in claims ⁻¹ or 2, wherein the oxirane is ethylene oxide.

5. A process as claimed in any one of the preceding claims, wherein the solvent is inert and essentially non-water-miscible.

6. A process as claimed in claim 5 wherein the solvent is an alcohol or ether according to formula (1)
R¹-O-R² (1)
in which R¹ is selected from hydrogen, a linear, branched, cyclic or aromatic C₁₋₂₀ hydrocarbyl or mono-or polyalkylene oxide, and R² is selected from a linear, branched, cylic or aromatic C₁₋₂₀ hydrocarbyl, alkoxy or mono- or polyalkylene oxide, or R¹, R² and O together form a cyclic ether.

7. A process as claimed in claim 5, wherein the solvent is an alcohol or ether according to formula (2) 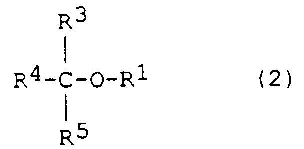 in which R¹ is selected from hydrogen or a C₁₋₈ hydrocarbyl and R³, R⁴ and R⁵ are independently selected from a C₁₋₈ hydrocarbyl, alkoxy or mono- or polyalkylene oxide.

8. A process as claimed in any one of the preceding claims, wherein the amount of hydroformylation catalyst is in the range of 0.01 to 1.0 wt% based on the weight of the reaction mixture.

9. A process as claimed in any one of the preceding claims, wherein the Group VIII metal is cobalt.

10. A process as claimed in any one of the preceding claims, wherein up to 10 mole% of the one or more Group VIII metal-based hydroformylation catalysts are phosphine-modified.

11. A process as claimed in any one of the preceding claims, wherein the one or more Group VIII metal-based hydroformylation catalysts are cobalt carbonyl compounds not modified by a phosphine.

12. A process as claimed in any one of the preceding claims, wherein the reaction mixture comprises a lipophilic promoter.

13. A process as claimed in claim 12, wherein the promoter is present in an amount in the range of 0.01 to 0.6 moles per mole of Group VIII metal.

14. A process as claimed in claims 12 or 13, wherein the lipophilic promoter is selected from sources of mono- and multivalent metal cations of weak bases; lipophilic mono-or dihydroxyarenes represented by formulae (3) and (4):
C₆R₅OH (3) C₆R₄(OH)₂ (4)
in which each R group is independently selected from hydrogen, a halide, a linear, branched, cyclic or aromatic C₁₋₂₅ hydrocarbyl, alkoxy or mono- or polyalkylene oxide, or in which two or more R groups together form a ring structure; lipophilic tertiary amines or arsines represented by formulae (5) and (6):
NR'₃ (5) AsR'₃ (6)
in which each R' group is independently selected from a C₁₋₂₅ linear, branched, cyclic and aromatic hydrocarbyl, alkoxy or mono- or polyalkylene oxide, or in which two or more of the R' groups together form a ring structure, and lipophilic phosphine oxides respectively arsine oxides represented by formulae (8) and (9):
O=PR"₃ (8) O=AsR"₃ (9)
in which each R" group is independently selected from a halide, a linear, branched, cyclic or aromatic C₁₋₂₅ hydrocarbyl, alkoxy or mono- or polyalkylene oxide, or in which two or more R groups together form a ring structure.

15. A process as claimed in claims 12 or 13, wherein the lipophilic promoter is selected from sodium acetate, phenol and nonylphenol, pyridine, 4- (1-butylpentyl)-pyridine, nonylpyridine, triphenylarsine and triphenylphosphine oxide.

16. A process as claimed in any one of the preceding claims, wherein the solvent contains water levels in the range of 1.0 to 2.5 wt%.

17. A process as claimed in any one of the preceding claims, wherein the oxirane is hydroformylated with hydrogen and carbon monoxide in a molar ratio within the range of 1:2 to 8:1.

18. A process as claimed in any one of the preceding claims, wherein the level of 3-hydroxyaldehyde in the reaction mixture is maintained at less than 15 wt%.

19. A process as claimed in any one of the preceding claims, wherein the hydroformylation is carried out at a temperature within the range of 50 to 100 °C and a pressure within the range of 3.5 to 34.6 MPa.

20. A process as claimed in any one of the preceding claims, wherein the hydroformylation product an aqueous liquid.

21. A process as claimed in claim 20, wherein the hydroformylation product is separated by extraction with water which is added to provide a water:mixture weight ratio within the range of 1:1 to 1:20.

22. A process as claimed in claim 20, wherein the Group VIII metal-based catalyst is recycled.

23. A process as claimed in any one of the preceding claims, wherein the 3-hydroxyaldehyde is hydrogenated to yield the 1,3-alkanediol.

## Patentansprüche

1. Verfahren zur Herstellung von 1,3-Alkandiolen und 3-Hydroxyaldehyden durch Hydroformylieren eines Oxirans mit Kohlenmonoxid und Wasserstoff in Gegenwart eines oder mehrerer Hydroformylierungskatalysatoren auf der Basis eines Gruppe VIII-Metalles, die bis zu 50 Mol%, bezogen auf das Metall, phosphinmodifizierte Katalysatoren enthalten können, und in Gegenwart eines organischen Lösungsmittels, worin die Konzentration des Oxirans zu Reaktionsbeginn weniger al 15 Gewichtsprozent (Gew.-%) beträgt, bezogen auf das Gewicht des gesamten flüssigen Reaktionsgemisches.

2. Verfahren nach Anspruch 1, worin die Konzentration des Oxirans weniger als 12 Gew.-% beträgt.

3. Verfahren nach den Ansprüchen 1 oder 2, worin das Oxiran ein Hydrocarbylepoxid mit 2 bis zu 30 Kohlenstoffatomen ist.

4. Verfahren nach den Ansprüchen 1 oder 2, worin das Oxiran Ethylenoxid ist.

5. Verfahren nach einem der vorstehenden Ansprüche, worin das Lösungsmittel inert und im wesentlichen mit Wasser unmischbar ist.

6. Verfahren nach Anspruch 5, worin das Lösungsmittel ein Alkohol oder Ether entsprechend der Formel (1)
R¹-O-R² (1)
ist, worin R¹ unter Wasserstoff, einem linearen, verzweigten, cyclischen oder aromatischen C₁₋₂₀ Hydrocarbyl oder Mono- oder Polyalkylenoxid ausgewählt ist und R² unter einem linearen, verzweigten, cyclischen oder aromatischen C₁₋₂₀ Hydrocarbyl, Alkoxy oder Mono- oder Polyalkylenoxid ausgewählt ist oder R¹, R² und O gemeinsam einen cyclischen Ether ausbilden.

7. Verfahren nach Anspruch 5, worin das Lösungsmittel ein Alkohol oder Ether entsprechend der Formel (2) 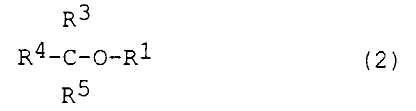 ist, worin R¹ unter Wasserstoff oder einem C₁₋₈ Hydrocarbyl ausgewählt ist und R³, R⁴ und R⁵ unabhängig unter einem C₁₋₈ Hydrocarbyl, Alkoxy oder Mono- oder Polyalkylenoxid ausgewählt sind.

8. Verfahren nach einem der vorstehenden Ansprüche, worin die Menge des Hydroformylierungskatalysators im Bereich von 0,01 bis 1,0 Gew.-%, bezogen auf das Gewicht des Reaktionsgemisches, liegt.

9. Verfahren nach einem der vorstehenden Ansprüche, worin das Gruppe VIII-Metall Cobalt ist.

10. Verfahren nach einem der vorstehenden Ansprüche, worin bis zu 10 Mol% des einen oder der mehreren Hydroformylierungskatalysatoren auf der Basis eines Gruppe VIII-Metalles phosphinmodifiziert sind.

11. Verfahren nach einem der vorstehenden Ansprüche, worin der eine oder die mehreren Hydroformylierungskatalysatoren auf der Basis eines Gruppe VIII-Metalles Cobaltcarbonylverbindungen sind, die nicht durch ein Phosphin modifiziert sind.

12. Verfahren nach einem der vorstehenden Ansprüche, worin das Reaktionsgemisch einen lipophilen Promotor umfaßt.

13. Verfahren nach Anspruch 12, worin der Promotor in einer Menge im Bereich von 0,01 bis 0,6 Mol je Mol Gruppe VIII-Metall zugegen ist.

14. Verfahren nach den Ansprüchen 12 oder 13, worin der lipophile Promotor unter Quellen für ein- und mehrwertige Metallkationen schwacher Basen; lipophilen Mono- oder Dihydroxyarenen, dargestellt durch die Formeln (3) und (4):
C₆R₅OH (3) C₆R₄(OH)₂ (4),
worin jede Gruppe R unabhängig unter Wasserstoff, einem Halogenid, einem linearen, verzweigten, cyclischen oder aromatischen C₁₋₂₅ Hydrocarbyl, Alkoxy oder Mono- oder Polyalkylenoxid ausgewählt ist oder worin zwei oder mehrere Gruppen R gemeinsam eine Ringstruktur ausbilden; lipophilen tertiären Aminen oder Arsinen, dargestellt durch die Formeln (5) und (6):
NR'₃ (5) AsR'₃ (6),
worin jede Gruppe R' unabhängig unter einem C₁₋₂₅ linearen, verzweigten, cyclischen und aromatischen Hydrocarbyl, Alkoxy oder Mono- oder Polyalkylenoxid ausgewählt ist oder worin zwei oder mehrere Gruppen R' gemeinsam eine Ringstruktur ausbilden; und lipophilen Phosphinoxiden beziehungsweise Arsinoxiden, dargestellt durch die Formeln (8) und (9):
O=PR"₃ (8) O=AsR"₃ (9),
worin jede Gruppe R" unabhängig unter einem Halogenid, einem linearen, verzweigten, cyclischen oder aromatischen C₁₋₂₅ Hydrocarbyl, Alkoxy oder Mono- oder Polyalkylenoxid ausgewählt ist oder worin zwei oder mehrere Gruppen R" gemeinsam eine Ringstruktur ausbilden, ausgewählt ist.

15. Verfahren nach den Ansprüchen 12 oder 13, worin der lipophile Promotor unter Natriumacetat, Phenol und Nonylphenol, Pyridin, 4-(1-Butylpentyl) -pyridin, Nonylpyridin, Triphenylarsin und Triphenylphosphinoxid ausgewählt ist.

16. Verfahren nach einem der vorstehenden Ansprüche, worin das Lösungsmittel Wasseranteile im Bereich von 1,0 bis 2,5 Gew.-% enthält.

17. Verfahren nach einem der vorstehenden Ansprüche, worin das Oxiran mit Wasserstoff und Kohlenmonoxid in einem Molverhältnis im Bereich von 1:2 bis 8:1 hydroformyliert wird.

18. Verfahren nach einem der vorstehenden Ansprüche, worin der Gehalt an 3-Hydroxyaldehyd im Reaktionsgemisch auf weniger als 15 Gew.-% gehalten wird.

19. Verfahren nach einem der vorstehenden Ansprüche, worin die Hydroformylierung bei einer Temperatur im Bereich von 50 bis 100°C und einem Druck im Bereich von 3,5 bis 34,6 MPa ausgeführt wird.

20. Verfahren nach einem der vorstehenden Ansprüche, worin das Hydroformylierungsprodukt eine wäßrige Flüssigkeit ist.

21. Verfahren nach Anspruch 20, worin das Hydroformylierungsprodukt durch Extraktion mit Wasser abgetrennt wird, das zur Ausbildung eines Gewichtsverhältnisses Wasser:Gemisch im Bereich von 1:1 bis 1:20 zugesetzt wird.

22. Verfahren nach Anspruch 20, worin der Katalysator auf der Basis eines Gruppe VIII-Metalles recycliert wird.

23. Verfahren nach einem der vorstehenden Ansprüche, worin der 3-Hydroxyaldehyd zur Ausbildung des 1,3-Alkandiols hydriert wird.

## Revendications

1. Procédé de préparation de 1,3-alcanediols et de 3-hydroxyaldéhydes par l'hydroformylation d'un oxiranne avec le monoxyde de carbone et l'hydrogène en présence d'un ou plusieurs catalyseurs d'hydroformylation à base de métaux du groupe VIII, qui peuvent contenir jusqu'à 50% molaires sur base du métal de catalyseurs modifiés par une phosphine et en présence d'un solvant organique, conformément auquel la concentration de l'oxiranne au démarrage de la réaction est inférieure à 15% en poids, sur base du poids du mélange réactionnel liquide total.

2. Procédé suivant la revendication 1, caractérisé en ce que la concentration de l'oxiranne est inférieure à 12% en poids.

3. Procédé suivant la revendication 1 ou 2, caractérisé en ce que l'oxiranne est un hydrocarbyl-époxyde possédant de 2 à 30 atomes de carbone.

4. Procédé suivant la revendication ⁻¹ ou 2, caractérisé en ce que l'oxiranne est l'oxyde d'éthylène.

5. Procédé suivant l'une quelconque des revendications précédentes, caractérisé en ce que le solvant est inerte et sensiblement non miscible à l'eau.

6. Procédé suivant la revendication 5, caractérisé en ce que le solvant est un alcool ou un éther répondant à la formule (1)
R¹-O-R² (1)
dans laquelle R¹ représente un atome d'hydrogène, un radical oxyde de mono- ou polyalkylène, ou un radical hydrocarbyle en C₁ à C₂₀, aromatique ou cyclique, linéaire ou ramifié et R² est choisi parmi un radical hydrocarbyle en C₁ à C₂₀ aromatique ou cyclique, linéaire ou ramifié, un groupe alcoxy ou oxyde de mono- ou polyalkylène, ou bien R¹, R² et O forment ensemble un éther cyclique.

7. Procédé suivant la revendication 5, caractérisé en ce que le solvant est un alcool ou un éther répondant à la formule (2) 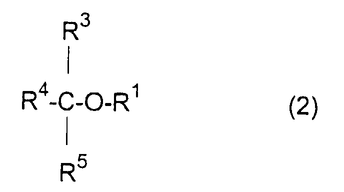 dans laquelle R¹ est choisi parmi l'hydrogène ou un groupe hydrocarbyle en C₁ à C₈ et R³, R⁴ et R⁵ sont indépendamment choisis parmi un radical hydrocarbyle en C₁ à C₈, un groupe alcoxy ou oxyde de mono- ou polyalkylène.

8. Procédé suivant l'une quelconque des revendications précédentes, caractérisé en ce que la quantité de catalyseur d'hydroformylation varie de 0,01 à 1,0% en poids par rapport au poids du mélange réactionnel.

9. Procédé suivant l'une quelconque des revendications précédentes, caractérisé en ce que le métal du groupe VIII est le cobalt.

10. Procédé suivant l'une quelconque des revendications précédentes, caractérisé en ce que jusqu'à 10% molaires d'un ou plusieurs catalyseurs d'hydroformylation à base de métaux du groupe VIII sont modifiés par une phosphine.

11. Procédé suivant l'une quelconque des revendications précédentes, caractérisé en ce qu'un ou plusieurs catalyseurs d'hydroformylation à base de métaux du groupe VIII sont des composés du type cobaltcarbonyle non modifiés par une phosphine.

12. Procédé suivant l'une quelconque des revendications précédentes, caractérisé en ce que le mélange réactionnel comprend un promoteur lipophile.

13. Procédé suivant la revendication 12, caractérisé en ce que le promoteur y est présent en une quantité qui varie de 0,01 à 0,6 mole par mole de métal du groupe VIII.

14. Procédé suivant la revendication 12 ou 13, caractérisé en ce que l'on choisit le promoteur lipophile parmi des sources de cations de métaux monovalents et polyvalents de bases faibles, des mono- ou dihydroxyarènes lipophiles représentés par les formules (3) et (4):
C₆R₅OH (3) C₆R₄(OH)₂ (4)
dans lesquelles chaque symbole R représente indépendamment un atome d'hydrogène, un atome d'halogène, un radical hydrocarbyle en C₁ à C₂₅ aromatique, cyclique, ramifié, linéaire, un radical alcoxy ou oxyde de mono- ou polyalkylène, ou dans lesquelles deux ou plus de deux groupes R ensemble forment une structure cyclique; des arsines ou des amines tertiaires lipophiles représentées par les formules (5) et (6) :
NR'₃ (5) AsR'₃ (6)
dans lesquelles chaque symbole R' représente indépendamment un radical hydrocarbyle en C₁ à C₂₅ aromatique, cyclique, ramifié ou linéaire, un radical alcoxy ou oxyde de mono- ou polyalkylène, ou dans lesquelles deux ou plus de deux des groupes R' forment ensemble une structure cyclique; et les oxydes de phosphines lipophiles et les oxydes d'arsines lipophiles, représentés par les formules (8) et (9) :
O=PR"₃ (8) O=Asr"₃ (9)
dans lesquelles chaque symbole R" est indépendamment choisi parmi un atome d'halogène, un radical hydrocarbyle en C₁ à C₂₅ aromatique ou cyclique, linéaire ou ramifié, un radical alcoxy ou oxyde de mono- ou polyalkylène, ou dans lesquelles deux ou plus de deux des symboles R forment ensemble une structure cyclique.

15. Procédé suivant la revendication 12 ou 13, caractérisé en ce que le promoteur lipophile est choisi parmi l'acétate de sodium, le phénol et le nonylphénol, la pyridine, la 4-(1-butylpentyl)pyridine, la nonylpyridine, l'oxyde de triphénylarsine et l'oxyde de triphénylphosphine.

16. Procédé suivant l'une quelconque des revendications précédentes, caractérisé en ce que le solvant contient des proportions d'eau qui varient dans la plage de 1,0 à 2,5% en poids.

17. Procédé suivant l'une quelconque des revendications précédentes, caractérisé en ce que l'oxiranne est hydroformylé avec de l'hydrogène et du monoxyde de carbone, dans le rapport molaire qui se situe dans la plage de 1:2 à 8:1.

18. Procédé suivant l'une quelconque des revendications précédentes, caractérisé en ce que la proportion de 3-hydroxyaldéhyde dans le mélange réactionnel est maintenue à une valeur inférieure à 15% en poids.

19. Procédé suivant l'une quelconque des revendications précédentes, caractérisé en ce que l'on entreprend l'hydroformylation à une température qui varie de 50 à 100°C et sous une pression qui varie de 3,5 à 34,6 MPa.

20. Procédé suivant l'une quelconque des revendications précédentes, caractérisé en ce que le produit de l'hydroformylation est un liquide aqueux.

21. Procédé suivant la revendication 20, caractérisé en ce que l'on sépare le produit d'hydroformylation par extraction par de l'eau que l'on ajoute pour obtenir un rapport pondéral eau:mélange qui varie de 1:1 à 1:20.

22. Procédé suivant la revendication 20, caractérisé en ce que le catalyseur à base de métal du groupe VIII est recyclé.

23. Procédé suivant l'une quelconque des revendications précédentes, caractérisé en ce que le 3-hydroxyaldéhyde est hydrogéné pour donner le 1,3-alcanediol.
